# EUROPEAN PATENT APPLICATION

(11) **EP 0 899 246 A1**
(43) Date of publication of application: **03.03.1999**
(21) Application number: 98113396.0
(22) Date of filing: 17.07.1998
(51) Int. Cl.: C04B 24/24, C04B 24/00

(54) **Phosphonocarboxylic acids and their use as cement setting retarders**

(30) Priority: 22.07.1997 GB 9715311; 06.06.1998 GB 9812141
(71) Applicant: ALBRIGHT & WILSON UK LIMITED, Oldbury, Warley, West Midlands B68 0NN (GB)
(72) Inventor: Davis, Keith Philip, Kinver, Staffordshire DY7 6BP (GB); Smith, Alan Craig, Bedworth, Warwickshire CV12 8TB (GB); Williams, Michael John, Bridgnorth, Shropshire WV15 4DR (GB)
(74) Representative: Savidge, Roger Gordon Madgwick

(57) **Abstract**

Phosphonocarboxylic acids and their salts are used to modify the setting of cement.

## Description

The present invention relates to setting modifiers for use in cement. The term "cement" is used herein broadly to include plaster and cement based products such as portland cement, hydraulic lime, pozzolan, slag, bauxite, high alumina refractories, ciment fondu, dental cements, both inorganic and resin based, gypsum, concrete, mortar, grout and fillers. In general cement refers to any particulate solid whose particles react when contacted with a liquid aqueous medium so as to bind together into a solid mass. The invention is primarily concerned with hydraulic cements and especially, high alumina cements.

Retarders are added to cements in order to prevent premature setting. Gypsum is conventionally added to portland cement to prevent flash setting, but adversely affects the strength of the product. The amount of gypsum that can be added is therefore strictly limited.

Retarders are required, for example, to prevent premature setting of ready mix concrete during transportation, or of oil well reinforcing cement during extended periods of pumping and at the high temperatures (e.g. up to 250°) which may be encountered downhole. Retarders are also required in dental cements to maintain plasticity while the filling is being shaped. They are frequently used to improve strength and to prevent overheating by the exothermic setting of concrete, especially during large scale civil engineering works such as dams.

Various retarders have been proposed. Among the compounds most commonly used are certain amino phosphonates. Typical of the latter are aminotris (methylenephosphonic) acid and its salts, and compounds of the formula M₂O₃PCH₂[NCH₂PO₃M₂ C₂H₄]ₙN(CH₂PO₃M₂)₂ where M is typically an alkali metal and n is an integer from 1 to five.

In addition to retardation it is often important to ensure even setting which requires dispersants and calcium scavengers to prevent migration of calcium and/or magnesium causing cracking of the surface. This is a particular problem with high alumina refractories.

A commonly used calcium scavenger in e.g. high alumina cements, is citric acid which is also a buffer to control pH but has the problem of forming citrate crystals.

Sodium polyacrylate is also often used in modifying cement setting as a dispersant which, however gives rise to severe problems of hygroscopicity.

A major problem with cement products arises when they are required to set while in contact with metals such as iron and steel. This situation is most commonly encountered in reinforced concrete. The problem concerns corrosion of the metal, which is one of the commonest causes of failure of reinforced concrete.

Retarding the setting of cement tends to increase the time during which any metal present is exposed to moisture, and hence to exacerbate problems of corrosion.

To overcome these various problems generally requires a number of additives such as retarders, corrosion inhibitors, dispersants, deflocculants, buffers and accelerators. Mixing these in the correct proportions under normal conditions of use often presents difficulties.

An object of the invention is to provide a novel setting retarder for cement which delays the completion of setting, and which preferably does not cause increased corrosion of any metal present. Another object is to provide a retarding agent which will retard the setting of, e.g. high alumina cements, will function as a dispersant but which is less hygroscopic than sodium polyacrylate, and which will replace citric acid as a calcium scavenger and buffer.

A particular object is to provide a setting retarder which both increases the set time of the cement and also reduces corrosion of any metal present. A further object is to simplify the preparation of cement by reducing the number of separate ingredients which are required.

We have now discovered that phosphonocarboxylic acids are highly effective as setting retarders which inhibit the corrosion of iron and steel and which are also effective dispersants, calcium scavengers, pH modifiers and deflocculants and which are less hygroscopic than sodium polyacrylate. The invention therefore consists in the use of phosphonocarboxylic acids or their salts as setting modifiers for cement.

Our invention provides according to one embodiment, a cement composition comprising particles which are capable of reacting in the presence of an aqueous liquid medium to form a coherent solid mass characterised by the presence of a phosphonocarboxylic acid or salt thereof in an amount adapted to modify the formation of said mass.

Our invention further provides articles and structures formed from the aforesaid composition. In particular, according to a further aspect, our invention provides articles and structures of metal reinforced concrete formed from the aforesaid cement composition, and sprayed high alumina refractories containing said phosphonocarboxylic acids and/or salts.

The preferred retarders for use according to the present invention are the water soluble salts of phosphonocarboxylic acids, especially the sodium and potassium salts. Other water soluble salts such as lithium or ammonium are less preferred, but we do not exclude the presence in the composition of alkaline earth metal salts such as calcium or magnesium. The retarder may be present at least partially as an aluminium salt.

For example 2-phosophono-1, 2, 4-tricarboxy butane and 2-hydroxy-2-phosphono acetic acid, respectively referred to herein as PTCB and HPA and their salts, have been found effective as have phosphonosuccinic acid and its salts. The most preferred phosphono carboxylates however are phosphonated telomers of active olefins which telomers have the formula

H[CHRCHR]ₙ-PO₃M₂

wherein at least one R group in each [CHR CHR] unit is a COOM group and the other R group is separately selected in each [CHR CHR] unit from hydrogen and COOM, hydroxyl, phosphono, sulphono, sulphato, C₁₋₇ alkyl, C₁₋₇ alkenyl groups and carboxylate, phosphono, sulphono, sulphato and/or hydroxy substituted C₁₋₇ alkyl and C₁₋₇ alkenyl groups, and each M is hydrogen or a cation and n is greater than 1, eg. up to
200. In the case of certain preferred telomers n is less than 100, especially less than 50, particularly less than 20, often less than 10, e.g. less than 6.

Also of value are salts of phosphonated cooligomers of the above formula, but in which the [CHRCHR]ₙ chain contains at least two [CHRCHR] groups derived from different monomers and in which n has a total value of at least 2. For example we include a phosphonated cooligomer of maleate and acrylate containing at least one [CH₂ CHCOOM] and at least one [CHCOOM CHCOOM] group.

The invention according to a preferred embodiment, uses a phosphonated oligomer or mixture of phosphonated oligomers of maleic or fumaric acid, of the formula:-H(CHCO₂M.CHCO₂M)ₙPO₃M₂ where n is from 1 to 6 and M is hydrogen or a cationic species and especially mixtures of such compounds with phosphonosuccinic acid or its salts, often in a minor proportion by weight based on the total weight of the solvent-free mixture. Preferably n has a value of less than 5, more preferably less than 3, especially 1.01 to 2, e.g. 1.1 to 1.8.

It is particularly preferred, according to the invention, to use reaction products which comprise: up to 50% by weight of a phosphonosuccinate based on the weight of the solvent-free composition; a phosphonated dimer of maleate; optionally a minor proportion by weight, relative to the weight of dimer, of higher phosphonated oligomers of maleate; and from 0.5 to 5% by weight of phosphate. A phosphonated oligomaleate reaction product of the above type having a degree of polymerisation of 1.2 to 1.5 will be referred to herein as POM.

The preferred retarders may be prepared in accordance with the teaching of GB-A-2252322 or EP-A-0569731, e.g. by reacting a salt of an unsaturated carboxylic acid with an alkali metal phosphite in an aqueous medium in the presence of a free radical initiator. In general any of the phosphono carboxylates described in the above specifications may be used in the invention. The preferred POM product is conveniently prepared by reacting sodium maleate with sodium phosphite in water at elevated temperature in the presence of a peroxy compound such as sodium persulphate or hydrogen peroxide, for instance as described in EP-A-0569731 Example 1.

Also preferred are products comprising from 50 to 99.5%, e.g. 90 to 99% by weight, phosphonosuccinic acid and minor proportions of dimer and optionally higher oligomers. Especially preferred are products obtained by phosphonating alkali metal fumarate with alkali metal phosphite at alkaline pH in an aqueous medium using a free radical initiator such as hydrogen peroxide.

The retarder is preferably present in the cement in a proportion of from 0.01 to 5% by weight active solids based on the total weight of solids, more preferably 0.02 to 2%, especially 0.05 to 1% eg 0.1 to 0.5%. The retarder is preferably dry mixed with the powdered cement as a finely divided powder e.g. less than 200 micron, desirably less than 100 micron, preferably less than 50 micron, most preferably less than 45 micron. Alternatively a solution of the retarder e.g. in water, may be added to the cement in the mixer. The solution may constitute all or part of the water required to form the slurry prior to setting.

The retarder may be added alone or in conjunction with other retarders, dispersants, deflocculants, buffers, synergists, accelerators or fillers.

The composition is preferably a hydraulic cement such as a portland cement, hydraulic lime, blast furnace slag or bauxite cement, masonry cement or ciment fondu typically made by calcining a mixture of limestone and clay. Alternatively the retarder may be used with a plaster, such as plaster of Paris, or with a dental cement, including resin based cements. The cement may be for use in the manufacture of reinforced concrete containing iron or steel reinforcement. A particular aspect of the invention provides use in high alumina cements.

The latter usually comprise calcium aluminate as their principal component. More generally they may typically comprise by weight between 10 and 50%, preferably between 25 and 45% CaO, between 50 and 90%, preferably between 55 and 75% Al₂O₃ and less than 10% SiO₂. They are typically prepared by calcining mixtures of limestone and bauxite, and are widely used for refractories. The invention is particularly applicable to sprayed high alumina refractory cements used in, for example, the steel industry.

We believe that the phosphonocarboxylic acids help to prevent or reduce migration of calcium and/or magnesium ions to the surface of the hardening cement, which migration causes the formation of brittle surface layers.

The invention is illustrated by the following examples :

### Example 1

Two samples were prepared comprising the following ingredients :
- 100: grams portland cement (unplasticized)
- 38: grams water
- 0.2: grams solids of POM

Two control samples were prepared containing cement only. All four samples were mixed in glass bottles. The water and any POM were mixed first. Then the cement was added, the bottle tightly closed and shaken to mix. One bottle was placed in an oven at 83°C and a duplicate was left at room temperature. The cement was inspected periodically by the use of a spatula. The cement was judged to be set when the spatula did not penetrate the surface.

Results are as follows :

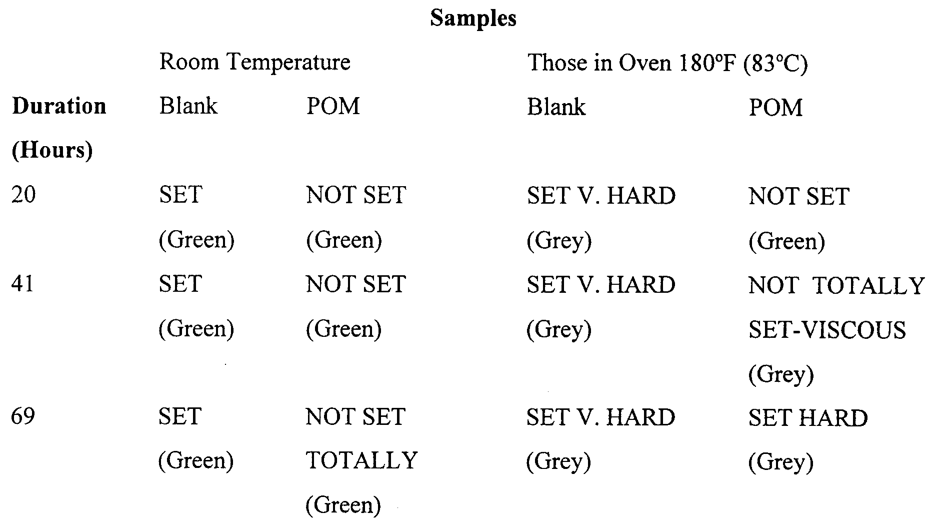

### Example 2

POM and PTCB were each used at a dose rate of 0.1% active acid to reduce the retardation time.

The pH of the water/phosphonate mix was adjusted to 12.5 pH with NaOH (ensuring that the total water + NaOH sln did not exceed 38g). Apart from this the details are the same as for the first test.

### Results of tests at room temperature:

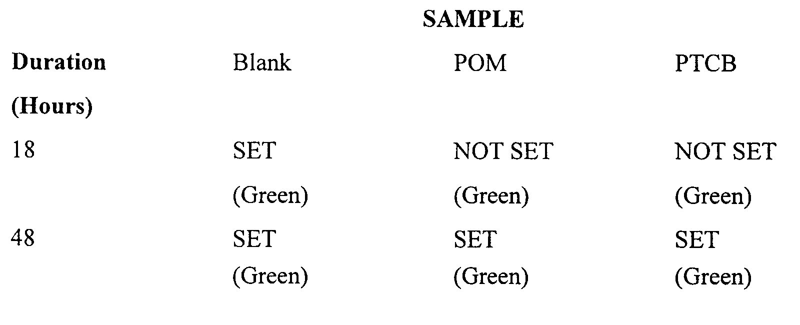

### Results of tests at elevated temperature (180°F): (83°)

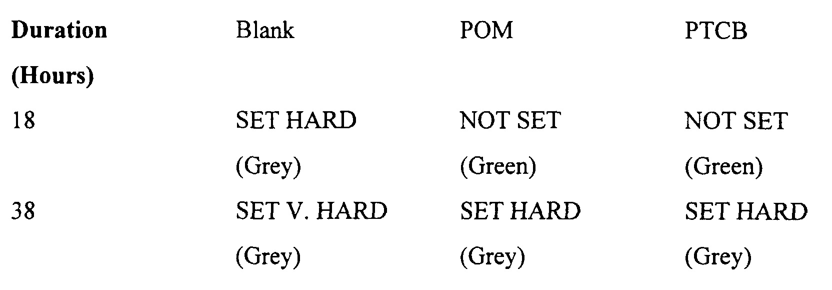

### Example 3

To 0.2g solids POM 38g water was added, then 100g calcium sulphate hemihydrate.

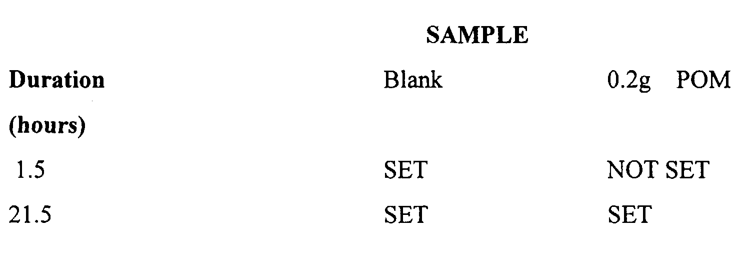

### Example 4

Using a glass polyalkenoate dental cement, two samples were prepared : One with POM, and one without. The approximate weights used are :

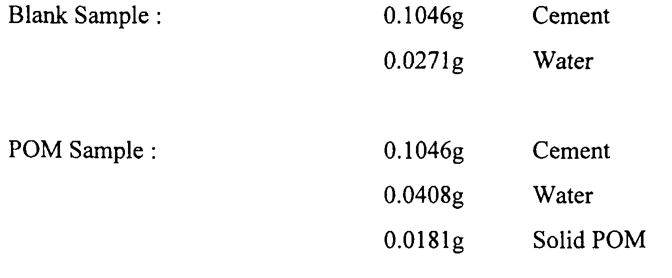

The curing time after mixing was measured until the cement balls became hard enough to have a comparable coefficient of restitution - where they could bounce off the table to the same degree (with the characteristic ringing sound).
- Blank: = 2 minutes setting time.
- POM sample: = 10 minutes setting time.

### Example 5

Mild steel electrodes were immersed in saturated lime solution containing 2% chloride having a pH greater than 12 and the corrosion was monitored at 25°C for 90 hours. Samples containing 1000 ppm active POM were compared with blanks. The composition of the samples was:
- Ca(OH)₂: 2gl⁻¹
- Ca C1₂ 2H₂0: 41.4gl⁻¹
- POM (as used): 1000ppm

The corrosion rate in mm per year based on three runs of 90 hours each was

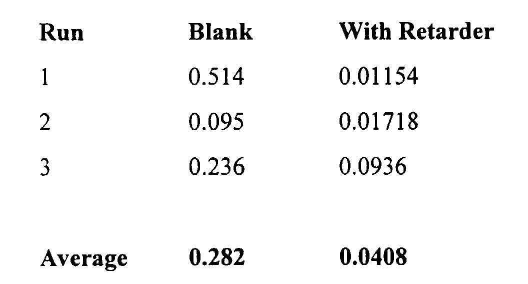

### EXAMPLE 6

POM is added to a high alumina refractory cement in a proportion of 0.22% by weight of POM based on the total weight of cement. The cement is sprayed and the setting characteristics and resistance to furnace temperatures is compared to those of the additive-free cement powder and of a commercial refractory cement containing sodium polyacrylate and citric acid as additives. The additive-free powder shows severe surface cracking and fails at high temperature. The commercial cement gives a crack-free finish which slows good temperature stability. The POM cement gives equivalent or superior performance to the commercial cement. The POM is less hygroscopic and more easily handled than the sodium polyacrylate. It avoids the problems of crystal formation often associated with citric acid and replaces two separate additions with a single addition.

## Claims

1. The use of phosphonocarboxylic acids or their salts to modify the setting of cement.

2. A cement composition comprising particles which are capable of reacting in the presence of an aqueous liquid medium to form a coherent solid mass characterised by the presence of a phosphonocarboxylic acid or salt thereof in an amount adapted to modify the formation of said mass.

3. A composition according to claim 2 comprising a portland or high alumina cement powder.

4. A composition according to claim 3 wherein said cement comprises from 25 to 45% lime, from 55 to 75% alumina and less than 10% silica, all based on the total weight of solids.

5. A composition according to any of claims 2 to 4 wherein said phosphonocarboxylic acid or salt thereof is present in a proportion of from 0.01 to 5% by weight based on the total weight of the solids in the composition.

6. A composition according to claim 5 wherein said phosphonocarboxylic acid or salt thereof is present in a proportion of from 0.05 to 1% based on the total weight of solids in the composition.

7. A composition according to any of claims 2 to 6 wherein said phosphonocarboxylic acid is selected from 2-phosphono-1,2,4-tricarboxylic acid, 2-hydroxy-2-phosphono acetic acid and phosphono succinic acid.

8. A composition according to any of claims 2 to 6 wherein said phosphonocarboxylic acid is a phosphonated telomer of an active olefin which telomer has the formula
H[CHRCHR]ₙ-PO₃H₂
wherein at least one R group in each unit is a COOH group and the other R group is separately selected in each unit from hydrogen and COOH, hydroxyl, phosphono, sulphono, sulphato, C₁₋₇ alkyl and C₁₋₇ alkenyl groups and n is greater than 1.

9. A composition according to any of claims 2 to 8 wherein said carboxylic acid is a phosphonated oligomer or mixture of phosphonated oligomers of maleic or fumaric acid, of the formula H(CHCO₂M.CHCO₂M)ₙPO₃H₂ where n is greater than 1.

10. A composition according to claim 9 wherein n is less than 6.

11. A composition according to claim 10 wherein n is from 1.01 to 2.

12. A composition according to any of claims 2 to 11 wherein the phosphonocarboxylic acid is added to said composition as its sodium or potassium salt.

13. A composition according to any of claims 2 to 12 in which said phosphonocarboxylic acid is present at least partially as a calcium, magnesium or aluminum salt.

14. A composition according to any of claims 2 to 13 in the form of an aqueous slurry.

15. A concrete structure made from a composition according to any of claims 2 to 14.

16. A structure according to claim 15 containing iron or steel reinforcement.

17. A refractory structure according to claim 15 formed by spraying a high alumina cement.
